**Europäisches Patentamt**

(19) **European Patent Office**

**Office européen des brevets**

(11) Publication number : **0 438 483 B1**

# (12) EUROPEAN PATENT SPECIFICATION

(45) Date of publication of patent specification :
**15.04.92 Bulletin 92/16**

(51) Int. Cl.⁵ : **C11C 3/08,** A61K 31/23

(21) Application number : **89911783.2**

(22) Date of filing : **10.10.89**

(86) International application number :
**PCT/DK89/00232**

(87) International publication number :
**WO 90/04008 19.04.90 Gazette 90/09**

(54) **TRIGLYCERIDES AND NUTRITIONAL COMPOSITION COMPRISING SUCH TRIGLYCERIDES.**

(30) Priority : **10.10.88 DK 5652/88**

(43) Date of publication of application :
**31.07.91 Bulletin 91/31**

(45) Publication of the grant of the patent :
**15.04.92 Bulletin 92/16**

(84) Designated Contracting States :
**AT BE CH DE FR GB IT LI LU NL SE**

(56) References cited :
**US-A- 4 753 963**

(73) Proprietor : **NOVO NORDISK A/S**
**Novo Allé**
**DK-2880 Bagsvaerd (DK)**

(72) Inventor : **HANSEN, Tomas, Tage**
**Tonedraget 5**
**DK-3450 Alleroed (DK)**
Inventor : **GODTFREDSEN, Sven, Erik**
**Smedegade 15B**
**DK-3500 Vaerloese (DK)**

(74) Representative : **Bach, Niels et al**
**c/o Novo Nordisk A/S Patent Dept. Novo Allé**
**DK-2880 Bagsvaerd (DK)**

EP 0 438 483 B1

## Description

The invention relates to triglycerides and a nutritional composition comprising such triglycerides.

Canola oil has a unique fatty acid composition. It consist of approx. 6% long chain saturated fatty acids, mainly palmitic acid. This make it the vegetable oil with the lowest amount of long chain saturated fatty acid, which is commercially available. As long chain saturated fatty acids increase blood coagulation, it is generally recommended to lower the amount of these fatty acids in our fat intake, as they are suspected to be connected with different cardiovascular disorder sickness, e.g. atherosclerosis. As a vegetable oil, it contain no cholesterol, which also is a big advantages, as cholesterol as well has been connected with increased risk of atherosclerosis. The two only essential fatty acids for man, i.e. the only ones the organism cannot synthesize, are α-linolenic acid and linoleic acid. All other fatty acids can be synthesized from these (Holman, R.T. et al., Nutrition Reviews, 40, 144.47 (1982)). They are the initial precursors for synthesis of prostaglandins. α-linolenic acid for the ω-3 type, linoleic acid for the ω-6 type. It is therefore necessary that both acids are present in any nutritional fat composition. For people on total parenteral nutrition (TPN), it is necessary to have them both present in the fat emulsion, or else the person will suffer from essential fatty acid deficiency diseases, which will have serious effects on constitution and fitness. Both acids are present in canola oil accounting for 10% and 26% respectively, making it the food oil with the highest content of α-linolenic acid. Many oils which have both fatty acids present have a high total amount of polyunsaturated fatty acids, making the oil oxidatively unstable. This is not the case with canola oil, which have a total of approx. 58% monounsaturated (oleic) acid, making the oil relatively stable against attack from oxygen. This is a big advantage, especially if the oil is formulated for parenteral nutrition. In such a case, a very fine emulsion is made having very small oil droplets and thereby a big surface area, which make the oil very vulnerable against oxidation. In this specification with claims "canola" will signify the corresponding mixture of acid residues from canola oil.

It is a well known fact, that triglycerides containing long chain fatty acids only are absorbed more slowly than the corresponding triglycerides containing medium chain length acids only ($C_8$-$C_{10}$) (Metabolism, Vol. 38, p. 507-513, 1989). Furthermore it is known that the human lipases, especially human pancreatic lipase, have a low activity towards polyunsaturated acids (Lipids, Vol. 22, 711-714, 1987) and in particular towards omega-3 fatty acids. This is the case also for the lipases as e.g. the lipoprotein lipase involved in cleavage of triglycerides applied as emulsions in parenteral nutrition.

Thus the purpose of the invention is the provision of triglycerides which can be formulated as a nutritional composition, and be used for either enteral or parenteral purpose, and when used for nutrition exhibits an improved bioavailability in regard to naturally occurring triglycerides containing canola oil acids.

A very important clinical aspect in regard to the bioavailability is the slower metabolism in comparison to emulsions containing MCT, which are known to cause metabolic acidosis, when infused intravenously.

The triglycerides according to the invention are 2-"canola"-1,3-didecanoyl glycerol.

Surprisingly it has been found that these triglycerides exhibit a better bioavailability of the long chain fatty acids from canola oil, which have so many beneficial nutritional properties as discussed previously. Furthermore, the triglycerides of the invention turn out, surprisingly, to be very efficient sources of energy in enteral as well as in parenteral nutrition, the long chain fatty acids of the triglycerides being in an optimal position in the molecule in respect to their cleavage by human lipases. Also, the triglycerides of the invention appear to allow preservation of the long chain fatty acids in a hydrophobic form which can pass the intestinal mucosal layer and reach the enteroside in an optimal form for further esterification, lipoprotein transport and clearance.

Surprisingly, the triglycerides of the invention appear to exhibit physical properties which allow facile formulation of the compounds in liquid products as well as in powdered products exhibiting excellent wettability properties. The hydrophobicity of the products of the invention as compared to other sources of canola acids presumably is of importance for this behaviour and properties of the compounds of the invention. In the liquid form the products of the invention possess excellent stabilities making sterilization of e.g. parenteral products containing the triglycerides of the invention reliable, easy and safe.

The triglycerides of the invention allow formulation of feeding products containing non-deteriorated canola acids including the essential and polyunsaturated acids which are sensitive to oxidation and polymerisation processes. Detrimental processes of these polyunsaturated fatty acids leading to negative nutritional forms are thus commonly associated with gastric and intestinal problems due to oxidation and polymerisation products of the polyunsaturated fatty acid. Such oxidized forms of canola acids can interfere with nitrogen uptake by interacting with sensitive amino acids in infant formula. These highly undesired effects are diminished or even avoided by applying triglycerides according to the invention.

For use in parenteral feeding the triglycerides according to the invention can be incorporated into lipid emulsions where the liquid phase amounts up to 30% of the emulsion and they can be processed using the usual techniques to provide chylomicronlike particles. The products of the invention turn out to exhibit physical properties particularly advantageous in regard to this application as compared to the known sources of canola acids. The triglycerides of the invention can advantageously be applied in such emulsions due to their fast conversion by lipoprotein lipase and endothelial lipase and the consequential avoidance of the discomfort and side effects of lipolipaedemia. Canola acids applied in triglycerides of the invention are thus cleared quickly and efficiently thereby providing the essential fatty acid concomitantly with short chain acids useful as energy substrates.

The use of triglycerides according to the invention in parenteral nutritional products is further particularly advantageous since the relatively high polarity of the triglycerides favour the stability of their emulsions which are subjected to severe heat treatments during their manufacturing. Use of such emulsion is particularly advantageous for nutrition of pre-term babies, elderly individuals and diseased patients for whom use of the conventional triglyceride emulsions as e.g. those based on unmodified canola oil offer a range of disadvantages. For example, supply of fat emulsions to these individuals over extended periods of time of the conventional products leads to an over-supply of canola acids relative to the energy supply of the conventional products which do not adequately reach certain tissues as e.g. the small intestine. In contrast, the products of the invention contain in a single molecular type of species a more appropriate balance between the canola acids and the short chain fatty acids which are suitable for supply of energy.

The use of triglycerides according to the invention for enteral and especially parenteral nutrition products is further particularly advantageous since the relatively high polarity of the triglycerides favour the stability of their emulsions which are subjected to severe heat treatments during their manufacturing. Use of such emulsion is particularly advantageous for critical ill patients on total parenteral nutrition as they both need energy (medium chain acids) and the long chain acids from canola oil as essential fatty acids.

A preferred embodiment of the triglycerides according to the invention is characterized by the fact that they have a purity of at least 30%, preferably at least 50%, more preferably at least 75%, and most preferably at least 90%. The higher the purity of the triglycerides, the more efficient the absorption of the triglycerides.

Also the invention comprises a nutritional composition, which comprises the triglycerides according to the invention. This composition can be either the triglycerides according to the invention without the other constituents which are necessary for a full nutritional composition, i.e. mainly vitamins, proteins and carbohydrates, or the triglycerides according to the invention together with these other constituents.

A preferred embodiment of the nutritional composition is parenterally administrable. This composition is an emulsion.

A preferred embodiment of the nutrional composition is enterally administrable. This composition is either an emulsion or an oil.

A preferred embodiment of the enteral composition according to the invention is in fluid form. The enteral composition can be an emulsion or a pure oil.

A preferred embodiment of the enteral composition according to the invention is in powder form, whereby the triglycerides are encapsulated. One of the manners, in which the droplets of triglyceride can be encapsulated, is described in Danochemo Technical Information on microencapsulated Product, Malmparken 5,2750 Ballerup, Denmark.

## Claims

1. The triglycerides 2-"canola"-1,3-didecanoyl glycerol.

2. Triglycerides according to Claim 1, which have a purity of at least 30%, preferably at least 50%, more preferably at least 75%, and most preferably at least 90%.

3. Nutritional composition, which comprises the triglycerides according to Claim 1 or 2.

4. Nutritional composition according to Claim 3, which is parenterally administrable.

5. Nutritional composition according to Claim 3, which is enterally administrable.

6. Nutritional composition according to Claim 5, which is in fluid form.

7. Nutritional composition according to Claim 5, which is in powder form, whereby the triglycerides are encapsulated.

## Patentansprüche

1. Die Triglyceride 2-"Raps (Canola)"-1,3-didecanoylglycerin.

2. Triglyceride nach Anspruch 1, gekennzeichnet durch eine Reinheit von wenigstens 30 %, vorzugsweise wenigstens 50 %, noch bevorzugter wenigstens 75 % und am bevorzugtesten wenigstens 90 %.

3. Nahrungszusammensetzung, die die Triglyceride nach Anspruch 1 oder 2 umfaßt.

4. Nahrungszusammensetzung nach Anspruch 3, dadurch gekennzeichnet, daß sie parenteral verabreichbar ist.

5. Nahrungszusammensetzung nach Anspruch 3, dadurch gekennzeichnet, daß sie enteral verabreichbar ist.

6. Nahrungszusammensetzung nach Anspruch 5, dadurch gekennzeichnet, daß sie in flüssiger Form vorliegt.

7. Nahrungszusammensetzung nach Anspruch 5, dadurch gekennzeichnet, daß sie in Pulverform vorliegt, wobei die Triglyceride gekapselt sind.

**Revendications**

1. Les triglycérides 2-« canola »-1,3-didécanoylglycérol.

2. Triglycérides selon la revendication 1 qui ont une pureté d'au moins 30 %, de préférence d'au moins 50 %, mieux d'au moins 75 %, et tout préférablement d'au moins 90 %.

3. Composition nutritionnelle qui comprend les triglycérides selon la revendication 1 ou 2.

4. Composition nutritionnelle selon la revendication 3 qui peut être administrée par voie parentérale.

5. Composition nutritionnelle selon la revendication 3 qui peut être administrée par voie entérale.

6. Composition nutritionnelle selon la revendication 5 qui est sous une forme fluide.

7. Composition nutritionnelle selon la revendication 5 qui est sous forme d'une poudre, les triglycérides étant encapsulés.